# EUROPEAN PATENT SPECIFICATION

(11) **EP 2 120 709 B1**
(45) Date of publication and mention of the grant of the patent: **13.07.2016**
(21) Application number: 08729481.5
(22) Date of filing: 11.02.2008
(51) Int. Cl.: A61B 5/103, B41J 3/36, B41J 3/407

(54) **SYSTEM AND METHOD FOR PROVIDING SIMULATED IMAGES THROUGH COSMETIC MONITORING**
SYSTEM UND VERFAHREN ZUR BEREITSTELLUNG SIMULIERTER BILDER DURCH KOSMETISCHE ÜBERWACHUNG
SYSTÈME ET PROCÉDÉ POUR LA FOURNITURE D'IMAGES SIMULÉES VIA SURVEILLANCE COSMÉTIQUE

(30) Priority: 11.02.2007 US 889292 P
(43) Date of publication of application: 25.11.2009
(73) Proprietor: TCMS Transparent Beauty LLC, Austin, TX 78746 (US)
(72) Inventor: IGLEHART, David, C., Austin, TX 78704 (US); EDGAR, Albert, D., Austin, TX 78727-6069 (US); YEAGER, Rick B., Austin TX 78759 (US)
(74) Representative: Conroy, John
(86) International application number: PCT/US2008/053528
(87) International publication number: WO 2008/098235

(56) References cited:
- WO-A-2007/022095
- US-A- 5 836 872
- US-A1- 2004 078 278
- US-B1- 6 554 452

## Description

### FIELD OF THE INVENTION

The current invention relates to automated computer-controlled methods to obtain digital images and to selectively and precisely apply one or more reflectance modifying agent, such as a dye or pigment, to human skin to improve its visual attractiveness.

### BACKGROUND OF THE INVENTION

### Prior Cosmetic Techniques and Their Disadvantages

Prior art techniques for modifying the appearance of skin include natural tanning, artificial tanning, and the deliberate application of cosmetics. Each of these prior art techniques has limitations.

Typically, the applications of cosmetic substances to skin are largely manual, for example through the use of brushes, application tubes, pencils, pads, and fingers. The application methods make prior art cosmetics imprecise, labor intensive, expensive, and sometimes harmful, when compared to the computerized techniques of the present invention.

Most prior art cosmetic approaches are based on the application of opaque substances. There is a need for the precise application of reflectance modifying agents (RMAs), such as transparent dyes, to provide a more effective modification of appearance.

Manual cosmetic applications are imprecise compared to computer-controlled techniques, and this imprecision may make them less effective. For example, the heavy application of a foundation base for makeup may cause an unattractive, caked-on appearance. Manual techniques also typically take a long time to employ, as can be seen in any morning commute on a highway, where people frantically take advantage of stops to finish applying their makeup. In addition, manually applied makeup is not cheap, and when the help of professionals such as beauticians is required, is even more expensive. Moreover, often the materials applied to the skin in manual techniques are themselves potentially harmful. For example, a foundation base for makeup may cause skin to dry out and may inhibit the skin's breathing. Sunlight or artificial light used for tanning may cause cancer.

Therefore, there is a need for the precise application of reflectance modifying agents (RMAs) to provide a more effective, more automated, faster, less expensive, and less dangerous modification of the appearance of skin. The cross-referenced U.S. patent application cited above presents a system and method for this need.

In this specification, the terms "reflectance modifying agent" or "RMA" refer to any compound useful for altering the reflectance of another material. Some examples of RMA are inks, dyes, pigments, bleaching agents, chemically altering agents, and other substances that can alter the reflectance of human skin and other features. The terms "dye" and "transparent dyes" are used for brevity in this specification to represent any RMA.

Consumers of cosmetics also need effective techniques that allow them to select desired cosmetic enhancements, such as different shades of makeup, to visualize how those enhancements will look on them, for example on computer displays, and to precisely apply cosmetics onto them to make the selected enhancements. Websites such as MatyKay.com offer virtual makeovers that allow users to try out on their home computer displays different makeup patterns on digital images of different types of women and even on digital images of the users themselves that users submit. However, the images used in virtual makeovers do not provide adequate details for the calculation of advanced RMA enhancements. Nor do they allow users to automatically apply to themselves the cosmetic enhancements that have selected digitally on computer displays. There is a need for a method that lets users employ an RMA applicator and a computer display to view sufficiently detailed digital images of themselves so that they can make virtual cosmetic enhancements to those images and so that they can automatically and precisely apply RMA to themselves to achieve those enhancements.

Consumers also need effective techniques that allow them to make cosmetic enhancements not just to a single area, such as a facial blemish, but over their whole bodies. For example, some people get natural or artificial tans to make their skin look smoother and thus more attractive over their whole bodies. Consumers in East Asia often use cosmetics to make much of their skin look lighter. Consumers also may want to make complex cosmetic enhancements, involving color and texture, to hide defects and enhance their appearance over their whole bodies. Manual techniques to make such whole body cosmetic enhancements can be particularly laborious, time-consuming, and expensive. There is a need for a system and method that lets users make automatic cosmetic enhancements to their whole bodies.

### Simulated Digital Images

In this patent application, a "simulated image" refers to a digital image that simulates a real object and can be displayed on a computerized device. A simulation of a real object is a portrayal of the object in any desired manner not strictly limited to aspects revealed by photographic or video data captured about the object. A simulated image can represent a still image of the object or a video clip of the object in motion and may be three dimensional (3D). For example, simulated images are widely used for display on computer screens, cell phones, video games, in animated sections of movies, and in medical imaging.

In general, consumers want to display very realistic simulated images in different media. Moreover, they may want simulated images that represent subjects that they choose. For example, these subjects may be the consumers themselves, their friends, their family members, or their favorite personalities such as movie stars. For instance, a boy may want to put his own face on a 3D action figure in a video game. A woman may want to display a simulated 3D image of her face and head on a computer device and make cosmetic enhancements to that image, so that she can try out different cosmetic effects and hairstyles virtually.

In addition, consumers may want simulated images that are enhanced to be more desirable in some way. For example, a person may want his or her own face to be displayed in a way that makes him or her appear younger and more attractive.

### Prior Techniques and Their Disadvantages

Simulated images have been created in a number of ways. They can be drawn by hand and then scanned, photographed, or video recorded and can be created through computer graphics programs, both of which are laborious techniques requiring special skills.

In addition, simulated images can be created by using sensors attached at various points to a real subject, digitally recording the motions the subject, often through multiple cameras, and using computer graphics programs to create simulated characters whose movements and facial expressions are based on those of the recorded subject. In this way, an animation of a cartoon character dancing or smiling may be based on a real actor's movements and expression.

The computer graphics programs used in these processes are increasingly able to transform recorded data about real objects into simulated images. An example is Optasia™, the model-based feature-recognition platform developed by Image Metrics, PLC. The "Technical White Paper" on the Image Metrics Website states that "The Optasia engine can perform rapid model-to-image matching regardless of the model type, including those with high-frequency elements such as texture." Optasia is available on a variety of platforms as a three-layered architecture. All systems use 1) the Optasia core, with 2) a sector specific API (e.g. medical image analysis). Prior knowledge is incorporated in the 3) 'expert' layer (or model).

However, such prior techniques have disadvantages:
- They are all labor intensive and require special skills and special, often cumbersome equipment not readily available to many consumers.
- The simulated images they produce have limited details so that they do not look as realistic as consumers want. Instead, they tend to look artificially smoothed, often because their computer graphics programs must fill in large areas of simulated images with what essentially amounts to guesses and averages as a result of limited collection of data about the subject. These guesses and averages may require complicated algorithms and large amounts of computing power. For example, the three-layered approach of Optasia, mentioned above, is complicated and computing intensive.
- They are difficult for consumers themselves to use to simulate favorite images.
- They are difficult for consumers to use to enhance simulated images in desirable ways.
- Moreover, they are not readily available to many consumers, who may not have access to expensive modeling and graphics software and equipment.

Therefore, there is a need for an automated cosmetic monitoring and enhancement system that can be readily available to consumers, is easy to use, and provides high-resolution realistic image files with rich data about real subjects that can be used for creating simulated images.

U.S. Patent Publication 2004/0078278 describes a cosmetic treatment method and device, in particular for care, make-up or colouring. A make-up or coloring method described in U.S. Patent Publication 2004/0078278 comprises steps which consist in imaging at least one part of the human body to be treated, analysing local characteristics of said part, and applying treatment products of said part based on said local characteristics.

U.S. Patent No. 5,836,872 describes a method for monitoring a region of a body surface includes recording at a first time a first multispectral digital image of the surface including the region, recording at a subsequent time a subsequent multispectral digital image of the surface including the region, and comparing the first and the subse quent images. Also, such a method in which the first and subsequent images are high magnification images, and further including recording low magnification images that include the high magnification images. Also, a method for forming a diagnostically useful classification of pigmented skin lesions described in U.S. Patent No. 5,836,872 includes using such a method to construct a database containing quantitatively extracted selected features from images recorded from a plurality of skin lesions, and correlating the features from each such lesion in the database with the medical history of the skin lesion from which the image was recorded. Also, a method for diagnosis of a premelanomatous or early melanomatous condition described in U.S. Patent No. 5,836,872 includes using the method for characterizing a surface region including the lesion and comparing the features of the lesion so obtained with the features in a database obtained from a number of skin lesions including lesions known to be premelanomatous or early melanomatous, or classifying the features of the lesion according to the diagnostically useful classification of pigmented skin.

U.S. Patent No. 6,554,452 describes an illumination method and apparatus for machine-vision systems, including a ring-light source (e.g., LEDs arranged in one or more circular rows), and a reflective-ring focusing element. The illumination source exhibits multi-directional ring-illumination properties which are useful for illumination of small components (which are being inspected or measured) without unwanted shadows. One embodiment described in U.S. Patent No. 6,554,452 provides a dark-field illumination system. One embodiment described in U.S. Patent No. 6,554,452 uses a strobed (or pulsed) power supply to drive the LEDs. Yet another embodiment described in U.S. Patent No. 6,554,452 uses a xenon strobe ring-light source and a backplane slit in place of the row of LEDs 25. In one such xenon strobe embodiment, a color filter, is also placed in series with the light path in order to obtain a monochromatic light. While xenon flashtube light sources tend to exhibit a five percent (5%) flash-to-flash variation in intensity which makes accurate measurements of certain characteristics difficult, they are useful in certain cases where intense white, or especially ultraviolet, light is desired. Strobed LEDs provide very little flash-to-flash variation in intensity. The compact ring-light generator has little, if any, shadowing.

### BRIEF SUMMARY OF THE INVENTION

These and other needs are addressed by the present invention. The following explanation describes the present invention by way of example and not by way of limitation. The invention defined by the subject-matter of the independent claim.

It is an aspect of the present invention to provide high-resolution realistic image files with rich data about real subjects that can be used for creating simulated images.

It is an aspect of the present invention to provide at least one wide-angle camera mounted on an RMA applicator to capture images ofa surface such as the human body.

It is another aspect of the present invention to provide a software method to use images captured by at least one wide-angle camera mounted on an RMA applicator to identify large features on a surface such as the human body.

It is another aspect of the present invention to provide a software method to use images captured by at least one high resolution camera mounted on the applicator head on an RMA applicator to identify small features on a surface such as the human body.

It is still another aspect of the present invention to provide a software method to use large and small features identified from captured images to create a map in computer memory of a surface such as the human body.

It is another aspect of the present invention to provide a software method to use a map in computer memory to track the location of an RMA applicator relative to an area of a surface such as the human body.

It is still another aspect of the present invention to provide at least one accelerometer mounted on an RMA applicator to identify changes in the acceleration of the applicator.

It is another aspect of the present invention to provide a software method to use images captured by at least one wide-angle camera mounted on an RMA applicator and data collected by an accelerometer mounted on the applicator to identify large features on a surface such as the human body.

It is another aspect of the present invention to provide a software method to use images captured by at least one high resolution camera mounted on the applicator head on an RMA applicator and data collected by an accelerometer mounted on the applicator to identify small features on a surface such as the human body.

It is another aspect of the present invention to provide a software method to use a map in computer memory and data collected by an accelerometer mounted on an RMA applicator to track the location of an applicator relative to an area of a surface such as the human body.

It is aspect of the present invention to provide a method for employing an RMA applicator and means to display images of a subject for cosmetic enhancement.

It is another aspect of the present invention to provide a method to let users select and display cosmetic enhancements of digital images of subjects.

It is still another aspect of the present invention to provide a method to let users automatically apply RMA to subjects to achieve cosmetic enhancement based on selections made to images on computer displays.

These and other aspects, features, and advantages are achieved according to the system and method of the present invention. In accordance with the present invention, an automated cosmetic applicator uses at least one wide-angle camera and at least one high resolution camera to capture images of a surface such as the human body. A process of differential lighting is used during capture of the images with the high-resolution camera to identify texture. The resulting images contain rich, pixel-level data about the subject's color, light value and texture characteristics. Software analyzes images to identify large and small features of the surface and to use these features as landmarks to create a corresponding map in computer memory. Software then uses this map to calculate cosmetic enhancements to aspects of the surface and to determine the position the applicator for accurate deposition of the cosmetics to achieve the enhancements. One or more accelerometers are added to the applicator to provide data that increases the accuracy of the map and of the positioning of the applicator. Software uses the map to provide "pix-rich" files, high-resolution realistic image files about the subject that are also data rich. Pix-rich files can be displayed and further modified for use as simulated still and video 3D images, for example in video games and animations.

In accordance with the present invention, an applicator head on a reflectance modifying agent (RMA) applicator scans an area on a user, such as a face. It uses software to sense aspects of color and texture, map the area, and automatically calculate cosmetic enhancements. One or more digital images representing the scan, mapping, and cosmetic enhancements are stored in computer memory. A user employs computer controls to view one or more images of the original scan and possible cosmetic enhancements on a computer display and to select and modify desired virtual cosmetic enhancements. Subsequently the user employs the applicator head on the RMA applicator to automatically apply RMA to the actual area to achieve the specified enhancements. During this process, landmarks on the area are used for registration to create the map of the area, to track virtual enhancements and to enable accurate positioning of the applicator head subsequently for precise application of the RMA to the area.

### BRIEF DESCRIPTION OF THE DRAWINGS

The following embodiment of the present invention is described by way of example only, with reference to the accompanying drawings, in which:
FIG. 1 is a block diagram that illustrates elements of an RMA applicator head and a computer with a display and controls;
FIG. 2A is a representative diagram that illustrates wide-angle cameras and accelerometers mounted on an RMA applicator;
FIG. 2B is a representative diagram that illustrates a top view of an RMA applicator showing the field of view of its wide-angle cameras;
FIG. 3 is a flow chart illustrating the general steps for determining the location of features on a surface such as the human body for mapping and the deposition of cosmetics;
FIG. 4 is a representative diagram illustrating a path of movement of the applicator head over an area of skin whereby multiple overlapping images may be captured;
FIG. 5 is a representative diagram illustrating aspects associated with the present invention that require registration; and
FIG. 6 is a flow chart illustrating the general steps for using the applicator head, computer display, and computer controls to select virtual cosmetic enhancements and apply RMA to achieve them on actual area.

### DETAILED DESCRIPTION OF EMBODIMENT - Determining head position by tracking large and small features

The referenced U.S. patent application No. 11/503,806 describes a computer-controlled system and method for scanning an area of human skin or other feature and automatically identifying unattractive attributes in that area, calculating cosmetic enhancements, and automatically applying RMA, typically through inkjet printing, to make those enhancements to that area.

The U.S. Patent Application No. 12/028,835 "HANDHELD APPARATUS AND METHOD FOR THE AUTOMATED APPLICATION OF COSMETICS AND OTHER SUBSTANCES" describes an applicator head with raised contact means, such as pounce wheels, for moving an RMS applicator over a surface.

One aspect of the present invention is the creation of rich image files that can be displayed and further modified for use as simulated still and video 3D images, for example in video games and animations. In this patent application, pix-rich™ files are files that contain rich, pixel-level data about a subject's color, light value and texture characteristics.

The present invention comprises innovations to the system and method that provide efficient tracking of the position of large features on the human body and smaller features on areas of skin and of the position of a cosmetic applicator relative to those features.

### Apparatus for Scanning and Applying Cosmetics

The following section explains one embodiment of an apparatus that scans a surface, calculates cosmetic enhancements, and applies reflectance modifying agents (RMAs) and that may also be used to provide data for pix-rich files.

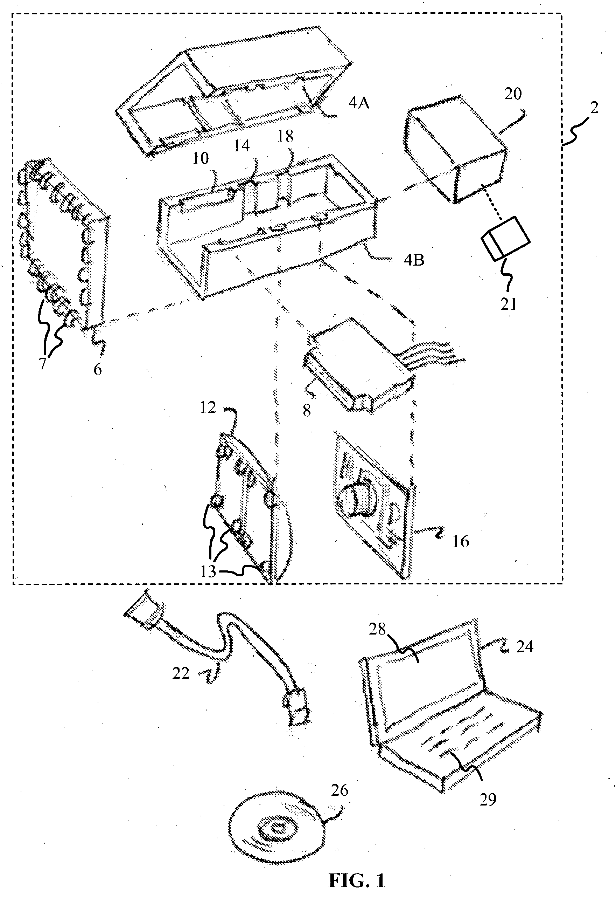

The applicator head **2**, shown in FIG. 1, covers an area of skin about equal to a single electric razor head. Such a size is proven daily to fit in intimate contact across a human face. In an embodiment for speed of application, multiple applicator heads **2** may be assembled in a floating mount, just as multiple floating heads are combined in a single electric razor.

### Elements

The applicator head **2** comprises the following elements.

### Plastic Case

The molded case **4A** and **4B** has rubber "O" type rings for waterproofing, so that the applicator head **2** can be run under the faucet for cleaning, like a razor. The inkjet printer head **8** can be maintained this way, which is not an option in normal printers. In an embodiment, the applicator head **2** may "park" for storage on a stand that would cap the applicator head **2**.

### Floating Ring

In one example, the applicator head 2 is moved across the skin by means of a floating ring 6 with pounce wheels 7, which are wheels with points around their outer rims.

The height of the points maintains a proper distance from the surface for both scanning and inkjet deposition. The pounce wheels **7** also reduce the amount of outside light entering around the base of the applicator to prevent distorting the accuracy of the scanning. In addition, the points on the pounce wheels **7** limit contact of the applicator head **2** with the cosmetics being deposited, to prevent smudging. Thus, they will typically leave behind minimal deposits of the RMA as they are moved over surfaces.

The pounce wheels **7** should be made of durable non-absorptive and hydrophobic material, for example silicon rubber or Teflon, so that they last and do not absorb the RMA. Their heights should also be low, for example 3/16 of an inch (4.8 mm). The use of low heights keeps the system close to the surface so that too much light does not come in underneath the system. The pounce wheels **7** may further be colored black to help absorb light. Their widths should be narrow to further reduce the area that comes into contact with the RMA. Their points should not be very sharp, so that they will not easily puncture surfaces such as skin.

In an embodiment, the pounce wheels **7** may be mounted on thin wires serving as axles.

In an embodiment, twelve pounce wheels may be mounted on each side of the floating ring **6**.

In an embodiment, a non-contact, electrostatic wipe (not shown) may be used to blow off the RMA from the pounce wheels **7**.

### Inkjet Head

A very thin inkjet head **8** fits perpendicularly to the skin into case groove **10**.

### Field Lens

A field lens **12** with LED assembly **13** provides telecentric viewing so that size is independent of distance and the view fits around the inkjet head. It fits into case groove **14** and helps protect the electronics behind the lens from water and dirt.

### Camera

A high-resolution camera module **16** with electronics fits into case groove **18**. In an embodiment, the high-resolution camera module **16** may be a module made for mobile devices such as cell phones. The newer of these modules have 3 megapixels and above. In covering an area half an inch across, just a 1 megapixel camera would have four times the resolution of the human eye at 10 inches (25 cm).

### Cosmetic Reservoir

A replaceable cosmetics reservoir **20** and ink is shown only as a block, but it should have a visually appealing and protectable design because it is what consumers would actually buy repeatedly, like razor blades. In an embodiment, the cosmetics reservoir **20** may contain multiple separate RMA colors that may be mixed to achieve desired effects. In another embodiment, it may contain a single RMA color premixed to achieve a desired aim color or effect.

### Attachments

### In an embodiment, the applicator head 2 is attached to the elements given below.

### Cable

In one embodiment, a data and power cable **22** is required. A USB 2.0 cable may be used.

### Computer

A consumer computer **24** is required. Almost any newer computer configured correctly with enough disk memory, good display, and a USB port may be used.

The computer **24** further comprises a display **28** and controls **29**, both known to those skilled in the art.

### Software

Software **26** is required that runs on the computer **24** and provides the functionality for scanning an area of a human feature, such as skin, calculating cosmetic enhancements, tracking registration, and applying the RMA, explained in detail in the cross-referenced application and outlined below.

In an embodiment, the software **26** also enables users to employ the RMA applicator head 2, the display **28**, and the controls **29** to view stored digital images of the scanned surface so that they can make virtual cosmetic enhancements to those images and so that they can automatically and precisely apply RMA to that surface to achieve those enhancements.

### Wide-Angle Cameras

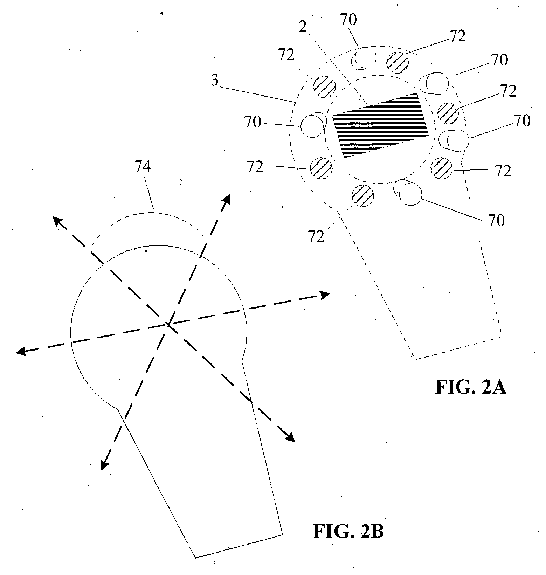

One or more wide-angle cameras **70**, shown in an embodiment in FIG. 2A, may be attached to the applicator **3**.

In an embodiment, six miniature wide-angle cameras 70 may be used, each wide-angle camera **70** covering a field of view **74**, shown in FIG. 2B, that is approximately 60° or more of the visual area below and around the applicator head **2**, so that 360° of the surface to be scanned is covered.

In this embodiment, the wide-angle cameras **70** are mounted on the side of the applicator **3**, around one or more applicator heads **2**, shown in FIG. 1, and look out and down, similar in topology to the eight eyes of a wolf spider, which provide vision around the spider. Wide-angle cameras **70**, shown in FIG. 2A, may thus be considered analogous to the visual element and are used for identifying the location of large features, such as bone structures, lips, and eyes, on the surface of the body and the position of the applicator **3** relative to that surface. Such slow movements may be characterized as providing low frequency data.

Some overlap in the images captures by the wide-angle cameras **70** may be useful for identifying the location of large features and the position of the applicator **3** relative to the surface.

In an embodiment, a camera may be focused on a mirror to expand the visual area of the camera. For example, a 360°mirror may enable a camera to operate as a wide-angle camera, as is known to those skilled in the art.

### Accelerometers

One or more accelerometers **72** may be used to measure the acceleration to give very precise tracking of rapid movements of the applicator **3**. Such rapid movements may be characterized as providing high frequency data. The use of accelerometers **72** enables tracking of the frame-by-frame location of the applicator **3**, relative to aspects of the surface being scanned.

For example, piezo accelerometers, known to those skilled in the art, may be used. In an embodiment, six piezo accelerometers **72** may be used around the sides of the applicator **3**, to provide data about movement along the X, Y, and Z axis of the applicator, representing pitch, yaw, and roll.

Accelerometers **72** may thus be considered analogous to an inner positioning sense, such as that of the human inner ear..

### Method of Operation

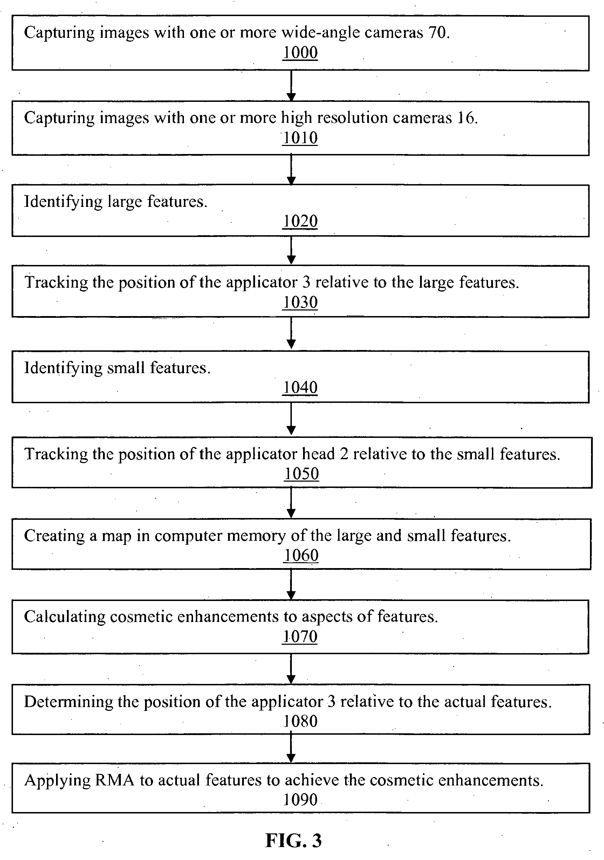

The present invention requires a complex software method to manage precision mapping of the whole body surface, similar to the problems that have been solved in gaming and modem animation. The general steps of this method are illustrated in FIG. 3.

### Step 1000 in FIG. 3 - Capturing images with one or more wide-angle cameras

In an embodiment, the user moves the applicator **3**, shown in FIG. 2A, inward toward the body to make contact with the skin, and the computer **24**, shown in FIG. 1, receives exposures representing an expanding view of the area of the surface, of the body from the wide-angle cameras **70**, shown in FIG. 2A. These exposures feed the computer **24**, shown in FIG. 1, landscape data showing the direction and proximity to large nearby features such as eyes, lips, breasts, and bone structures such as knees, and monitors the disposition and motion of limbs. After the applicator head **2**, shown in FIG. 1 makes contact with the skin, the wide-angle cameras **70**, shown in FIG. 2A, continue to feed the computer this landscape data about large features.

The wide-angle cameras **70** thus act analogously to the sensors on a Lunar Lander, which map large landmarks on the surface of the moon.

### Step 1010 in FIG. 3 - Capturing images with one or more high resolution cameras

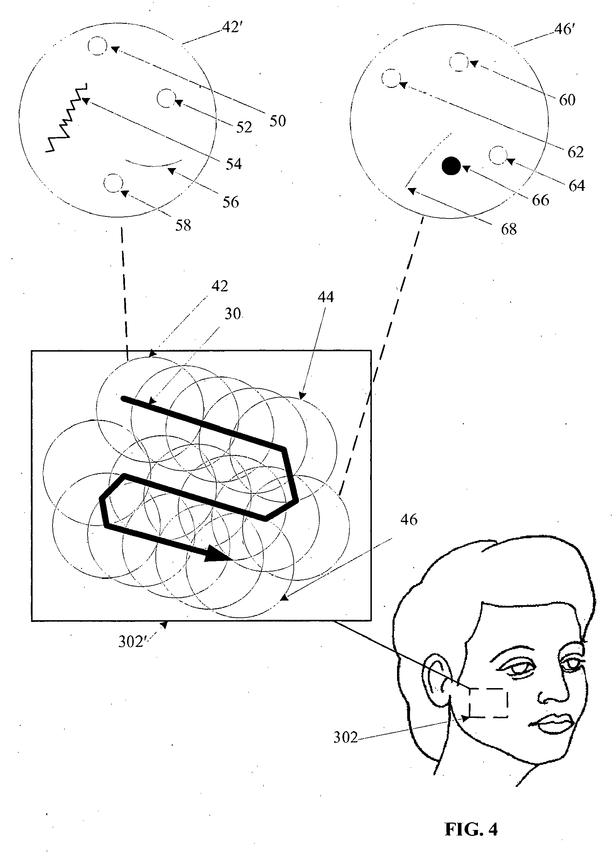

In an embodiment, after the applicator head **2**, shown in FIG. 1, makes contact with the skin, the user moves the applicator head **2**, anywhere over the skin, much like using an electric razor. For example, the user may move the applicator head **2** over an area of skin **302**, shown in FIG. 4, in path of movement **30**.

The camera module **16**, shown in FIG. 1, then captures overlapping images, preferably at least 10 per second, from the area of skin **302**, shown in FIG. 4, under applicator head **2**, shown in FIG. 1. Most of the image at each capture is redundant with the previous capture. For example, images **42**, **44**, and **46**, shown in FIG.4, are captured.

In an embodiment, an audible signal from a sound source, for example buzzer **21**, shown in FIG. 1, guides the user to completion of each area,

In another embodiment, the motion and action of the applicator head **2** is tracked on the computer display **28**.

In another embodiment, the user always starts an applicator session at a fixed point, such as the top center of the forehead, waits for a buzz sound to confirm acquisition of data, and scans from the touchdown point in a defined pattern, for example back and forth left and right moving the applicator head 2 down about 1/8 width on each pass. In this way, software **26** has the simplified task of staying locked on position rather than tracking complex and arbitrary non-overlapping movements.

### Step 1020 in FIG. 3 - Identifying large features

In an embodiment, the software **26**, shown in FIG. 1, analyzes the exposures from the wide-angle cameras **70**, shown in FIG. 2A, in comparison with at least one generic map of large human features previously stored in computer memory, for example in the non-volatile memory of computer **24**, shown in FIG. 1.

Such generic maps of human features are used in the gaming and animation industries, known to those skilled in the art. An example is Optasia™, the model-based feature-recognition platform developed by Image Metrics, Plc. The "Technical White Paper" on the Image Metrics website states that, "The Optasia engine can perform rapid model-to-image matching regardless of the model type, including those with high-frequency elements such as texture."

Thus, software **26** can identify the location of large features from the data received from the wide-angle cameras **70**, shown in FIG. 2A.

### Step 1030 in FIG. 3 - Tracking the position of the applicator relative to the large features

Software **26**, shown in FIG. 1, also calculates the position of the applicator 3, shown in FIG. 2A, in relation to the location of the large features that it has identified.

A visual position sense, resulting from the wide-angle cameras **70**, shown in FIG. 2A, is accurate at low frequencies. In fact, because averaging can be used, it is more accurate at low frequencies. The wide-angle cameras **70** can find position and monitor very slow movements accurately.

### Using Accelerometers

In an embodiment comprising one or more accelerometers **72**, shown in FIG. 2A, the software **26**, shown in FIG. 1, further calculates the position of the large features and of the applicator **3**, shown in FIG. 2A, based on rapid movements of the applicator **3**.

In this tracking process, all the sensors thus continuously refine calibration during each applicator session as the precision motion measured by visual trains the gain and offset of the sensors. This is the relationship between the human visual and positional sensors.

An accelerometer **72**, shown in FIG. 2A, is useless at finding absolute position (DC), but it can accurately track fast movements, up to audio frequencies at which visual is useless, and in fact can be used as a microphone. In between, there is a crossover frequency for which the two measurements, from the wide-angle cameras **70** and from the accelerometers **72**, can be crossed over, like a woofer and tweeter. Around this frequency range the visual position is used to adjust and continuously refine the gain and offset of the accelerometer **72**. In the applicator **3**, this crossover may be at around 1 Hz, in an embodiment.

### Step 1040 in FIG. 3 - Identifying small features

Software **26**, shown in FIG. 1, analyzes the captured images from the high-resolution camera **16**, such as images **42**, **44**, and **46**, shown in FIG. 4, and identifies landmarks, or "skinmarks" in those images. Many aspects of human features may be used as skinmarks, for example, pores, moles, scars, lines, wrinkles, age spots, sun damage, freckles, color variations, contours of features, and textural variations such as bumps. For example, an expanded view **42'** of captured image **42** represents a distinctive pattern of skinmarks: a pore **50**, another pore **52** in a different location, a scar **54**, a wrinkle **56**, and a third pore **58** in yet another location. Expanded view **46'** of captured image **46** represents a different distinctive pattern: a pore **60**, another pore **62**, a third pore **64**, a mole **66**, and a wrinkle **68**.

In general, each type of skinmark has distinctive characteristics that can be identified from the scanned data, based on empirical studies of scanned skinmarks. Examples of such characteristics are explained in the cross-referenced patent application.

In an embodiment, an applicator **3**, shown in FIG. 2A, may comprise multiple applicator heads **2**, shown in FIG. 1, and so employ multiple high-resolution cameras **16** to capture images that can be analyzed to identify skinmarks.

### Step 1050 in FIG. 3 -Tracking the position of the applicator head relative to the small features

The positional information provided by the skinmarks described above enables the software **26**, shown in FIG. 1, to keep the applicator head **2**, the area of skin **302**, shown in FIG. 4, and computer models in register. Thus, whenever the applicator head **2**, shown in FIG. 1, is placed on the skin, software **26** can track where applicator head **2** is located relative to the skinmarks on a global map, described below, while the global map is being assembled, using the skinmarks as they are identified, and after the whole map, containing all the skinmarks, has been completed.

In a worst case scenario, the software **26** may have to process a large area of the skin to find a non-redundant set of skinmarks for tracking.

### Using Accelerometers

In an embodiment comprising one or more accelerometers **72**, shown in FIG. 2A, the software **26**, shown in FIG. **1**, further calculates the position of the small features and of the applicator head **2** based on rapid movements of the applicator **3**, shown in FIG. 2A.

As mentioned above, in this tracking process all the sensors thus continuously refine calibration during each applicator session as the precision motion measured by visual trains the gain and offset of the sensors. This is the relationship between the human visual and positional sensors.

### Step 1060 in FIG. 3 - Creating a map in computer memory of the large and small features

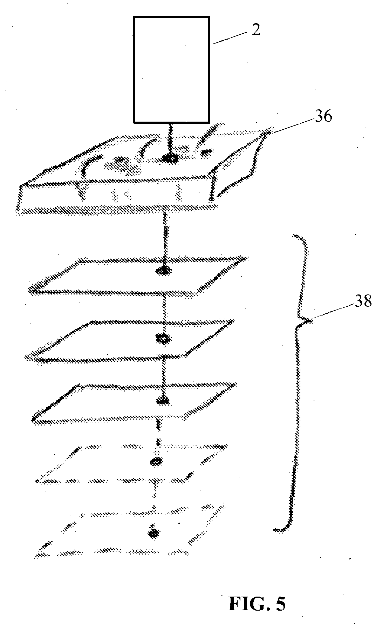

As shown in FIG. 5, the method of the present invention requires knowledge of applicator head **2** position relative to real skin **36** and other features and a mapping from real skin **36** and other features to abstract layers **38** in computer memory that
- model that skin and other features,
- describe aesthetic choices,
- guide execution strategies, and
- track long-term changes in the appearance of the skin and other features.

Software **26**, shown in FIG. 1, uses the large and small features it has identified relative to the position of the applicator **3**, shown in FIG. 2A, to assemble a global map of the scanned surface and store it in computer memory, for example on computer **24**, shown in FIG. 1. Moreover, the global map can be continually revised each time the applicator **3**, shown in FIG. 2A, is moved to scan an additional area of skin or other feature, so that a global map of the entire desired area of the skin can be assembled and revised through further scanning.

In an embodiment, the global map is stored automatically at periodic intervals. In another embodiment, it is stored each time the applicator head **2**, shown in FIG. 1, is raised from the surface being scanned. Thus, the global map enables registration of applicator position, scanned surface, and computer strategy layers through time between applications sessions and even days.

If given the picture of a city, including many streets, houses, and trees, one could track where in the city the area was, even if some of the houses changed and the leaves changed color for autumn. In a similar way, changes in human features such as living skin, for example color changes and the appearance of new wrinkles, do not impede the accuracy of overall tracking through the global map.

In an embodiment, the computer map tracks the skeletal structure of a human body, bone by bone and joint by joint, from images captured by the wide-angle cameras **70**, shown in FIG. 2A. Small features on the surface of the skin are referenced by their position relative to underlying bone structure, in whatever way that bone structure moves, so that a frexel, or area of skin, keeps a constant position relative to this complex system even during active motion of the body.

### Frexels

In this patent specification, the term "frexel" is defined as a small pixel-like region of the skin. A frexel might correspond to a small portion of a freckle or other skin feature, or it may correspond to an area of the skin that does not have special features. A frexel thus refers to skin rather than to an independent coordinate system. The term frexel is used to suggest that what is being measured is on a 3-D surface rather than a flat surface.

### Step 1070 in FIG. 3 - Calculating cosmetic enhancements to aspects of features

### Automatic Enhancements

In an embodiment, the software **26**, shown in FIG. 1, can automatically calculate and, in an embodiment display, cosmetic enhancements to the features using the methods described in the cross-referenced patent application. For example, these enhancements can be for smoothing skin, lightening skin, camouflaging blemishes, and making freckles crisper.

In addition, the software **26** can carry out overall enhancements based on input by designers' looks, like an evening look or a Nicole Kidman look. One embodiment of this technique is described in the referenced provisional patent applications.

These automatic enhancements may be done automatically or in response to user selections.

### User Input

In an embodiment, the user can specify all or part of the enhancements, including modifications of automatic enhancements.

### Step 1080 in FIG. 3 - Determining the position of the applicator relative to the actual features

After the enhancements have been calculated, the user again moves the applicator **3**, shown in FIG. 2A, over the surface to be cosmetically enhanced. The wide-angle cameras **70**, shown in FIG. 2A, and the high-resolution camera **16**, shown in FIG.1, again capture images of the surface.

Software **26** again identifies large and small features of the scanned surface and the position of the applicator **3**, shown in FIG. 2A, relative to landmarks and skinmarks in the global map of the scanned surface. Thus, software **26** can determine the position of the applicator relative to actual features.

In an embodiment comprising one or more accelerometers **72**, shown in FIG. 2A, the software **26**, shown in FIG. 1, further calculates the position of the large and small features and of the applicator **3**, shown in FIG. 2A, based on rapid movements of the applicator **3**.

### Step 1090 in FIG. 3 - Applying RMA to actual features to achieve the cosmetic enhancements

When the software **26**, shown in FIG. 1, determines that an area of skin or other feature for which an enhancement has been specified is underneath the inkjet head **8**, it instructs the inkjet head **8** to apply the RMA, contained in the cosmetic reservoir **20**, to accomplish the enhancement.

In an embodiment, the RMA can be deposited on multiple passes of the applicator head **2** over the skin until the chosen enhancement is accomplished. Then no more RMA is deposited on subsequent passes.

The entire process of scanning, making virtual enhancements, and applying RMA may be repeated to the user's satisfaction.

### DETAILED DESCRIPTION - Creating Pix-Rich Files

In an innovation to the system and method described above, the detailed, highly accurate global maps created for cosmetic enhancements are saved as pix-rich files. These pix-rich files may then be moved to any computer device and used as digital images for any virtual display, for example in gaming, animation, cell phone, and medical applications. For example, the planes of data in the global maps may be saved, partially or as a whole, as compressed JPEGF files, known to those skilled in the art.

### Richer, More Manipulable Data for Realistic Simulations

Pix-rich files are a new medium because the data they provide about a scanned surface is much richer in positional information, reflectance (RGB) and topographical (texture) data than the data in previous files of scanned images or photographs. For example, the technique of differential lighting, explained in the cross-referenced patent applications, provides greatly increased data about the topology of a scanned surface, showing more information about large and small features, from eyes and cheekbones down to the level of pores in the skin. In addition, more data is obtained because many different frames of scanned data about small pieces of the surface and are the put together to assemble the global maps. This further provides very high resolution data about the surface.

As a result, pix-rich files can provide much more realistic images of people for simulated images, greatly reducing the problem of over-smoothness.

Because they provide richer data, they are also dynamically manipulable electronically. That is, they are much easier to manipulate by software, such as gaming or animation applications, because such software does not have guess and calculate for as much missing information or work as hard to recognize feature to create simulate images.

### Availability

Pix-rich images will be potentially much more available to uses through home scanning embodiments of the cosmetic scanning and application system described above.

### User Enhancements

Users will be able to use home scanning embodiments of the cosmetic scanning and application system described above to cosmetically enhance images of themselves that can be saved as pix-rich files. This will help users be able to present simulated images of themselves as they want to be seen.

### DETAILED DESCRIPTION - Alternate Embodiments

### Other Hardware Configurations

The applicator head **2**, shown in FIG. 1, is attached to a separate computer **26** with a display **28** and controls **29**. In another embodiment, the applicator head **2** may comprise an element of an RMA applicator that itself is a computerized device with non-volatile memory, a display, and controls, so that no separate computer is required as an attachment.

### Application of Other Substances than RMAs

The applicator head 2 of the present invention may be used to apply other substances than RMAs, for example medically beneficial compounds or live skin.

### Application to Other Area than Skin

The applicator head 2 of the present invention may be used to apply RMAs and other substances to other areas than human skin. For example, it may apply substances to fabrics, fruit, walls, leaves, and flowers.

### Other Uses for Global Maps

The detailed, highly accurate global maps created for cosmetic enhancements may be used as digital images for any virtual display, for example in gaming, animation, cell phone, and medical applications.

### DESCRIPTION OF EMBODIMENT - Whole body cosmetic enhancement

### Method of Operation

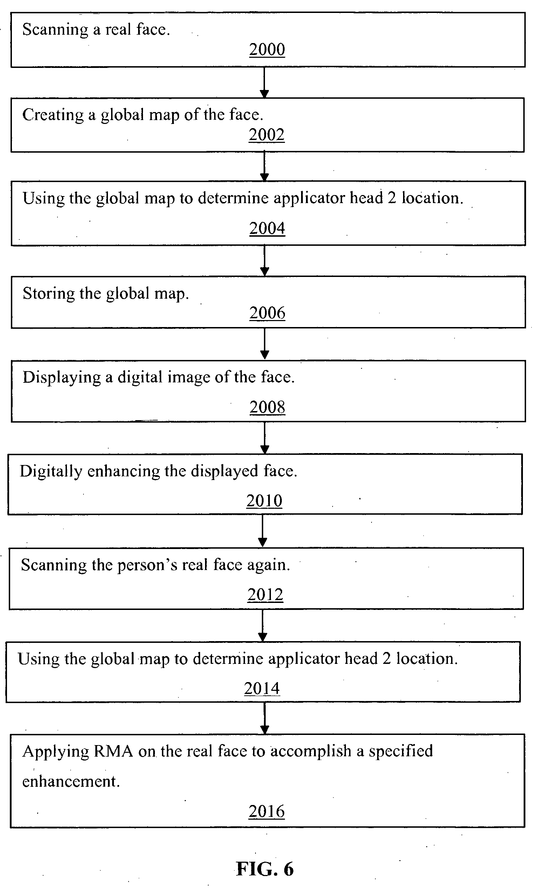

A typical use of the present invention is for making cosmetic enhancements to a person's face, but analogous enhancements could be made to any other area of the human body or other surfaces. In the explanation of the present invention's method given below, the face is use as a specific example and further represents any area of human skin, any human feature, or any other surface to be enhanced. The general steps of this method are illustrated in FIG. 6.

### Step 2000 in FIG. 6 - Scanning a real face.

In an embodiment, the user moves the applicator head **2**, shown in FIG. 1, anywhere over the face, very much like using an electric razor. For example, the user may move the applicator head **2** over an area of skin **302**, shown in FIG. 4, in path of movement **30**. The face may be that of the user or any one else.

The camera module **16**, shown in FIG. 1, then captures overlapping images, at least **10** per second, from the area of skin **302**, shown in FIG. 4, under applicator head **2**, shown in FIG. 1. Most of the image at each capture is redundant with the previous capture. For example, images **42**, **44**, and **46**, shown in FIG. 4, are captured.

In an embodiment, an audible signal from a sound source, for example buzzer **21**, shown in FIG. 1, guides the user to completion of each area,

In another embodiment, the motion and action of the applicator head **2** is tracked on the computer display **28**.

In another embodiment, the user always starts an applicator session at a fixed point, such as the top center of the forehead, waits for a buzz sound to confirm acquisition of data, and scans from the touchdown point in a defined pattern, for example back and forth left and right moving the applicator head **2** down about 1/8 width on each pass. In this way, software **26** has the simplified task of staying locked on position rather than tracking complex and arbitrary non-overlapping movements.

### Step 2002 in FIG. 6 - Creating a global map of the face.

As shown in FIG. 5, the method of the present invention requires knowledge of applicator head **2** position relative to real skin **36**, and a mapping from real skin **36** to abstract layers **38** in computer memory that model that skin, describe aesthetic choices, guide execution strategies, and track long-term changes in the appearance of the skin.

To track positioning, the software **26** analyzes the captured images of the face, such as images **42**, **44**, and **46**, shown in FIG. 4, and identifies landmarks, or "skinmarks" in those images. Many aspects of human features may be used as skinmarks, for example, pores, moles, scars, lines, wrinkles, age spots, sun damage, freckles, color variations, contours of features, and textural variations such as bumps. For example, an expanded view **42'** of captured image **42** represents a distinctive pattern of skinmarks: a pore **50**, another pore **52** in a different location, a scar **54**, a wrinkle **56**, and a third pore **58** in yet another location. Expanded view **46'** of captured image **46** represents a different distinctive pattern: a pore **60**, another pore **62**, a third pore **64**, a mole **66**, and a wrinkle **68**.

Software **26**, shown in FIG. 1, identifies such distinctive patterns of skinmarks in each captured image, such as **42**, **44**, and **46**, shown in FIG. 4, in an area of skin **302** and uses them to assemble a global map of the face. Moreover, the global map can be continually revised each time the applicator head **2** is moved to scan an additional area of skin or other feature, so that a global map of the entire desired area of the face can be assembled and revised through further scanning.

### Step 2004 in FIG. 6 - Using the global map to determine applicator head 2 location.

The positional information provided by the skinmarks described above enables the software **26**, shown in FIG. 1, to keep the applicator head **2**, the area of skin **302**, shown in FIG. 4, and computer models in register. Thus, whenever the applicator head **2** is placed on the face, software **26**, shown in FIG. 1, can track where applicator head **2** is located relative to the skinmarks on the global map, while the global map is being assembled, using the skinmarks as they are identified, and after the whole map, containing all the skinmarks, has been completed.

In a worst case scenario, the software **26** may have to process a large area of the face to find a non-redundant set of skinmarks for tracking.

### Step 2006 in FIG. 6- Storing the global map.

An innovation is that software **26**, shown in FIG. 1, stores the global map in non-volatile memory on the computer **24**. In an embodiment, the global map is stored automatically at periodic intervals. In another embodiment, it is stored each time the applicator head **2** is raised from the face.

Thus, the global map enables registration of applicator head **2** position, skin, and computer strategy layers through time between applications sessions and even days.

If given the picture of a city, including many streets, houses, and trees, one could track where in the city the area was, even if some of the houses changed and the leaves changed color for autumn. In a similar way, changes in living skin, such as color changes and the appearance of new wrinkles, do not impede the accuracy of overall tracking through the global map.

### Step 2008 in FIG. 6 - Displacing a digital image of the face.

After the global map has been stored, a user can use the computer controls **29**, shown in FIG. 1, to display a high level, representative image of the face on the computer display **28**. The displayed image is a richly detailed digital image.

### Step 2010 in FIG. 6 - Digitally enhancing the displayed face.

The user can then employ the computer controls **29**, shown in FIG. 1, to make virtual cosmetic enhancements to the displayed image of the face, for example to change areas of coloring or to achieve an overall "look." Software **26** correlates the virtual enhancements with the appropriate skinmarks in the global map.

In different embodiments, the virtual enhancements can be made automatically and through specific user input.

### Automalic Enhancements

In an embodiment, the software **26** can automatically calculate and display virtual cosmetic enhancements to the area of skin **302** using the methods described in the cross-referenced patent application. For example, these enhancements can be for smoothing skin, lightening skin, camouflaging blemishes, and making freckles crisper.

In addition, the Software **26** can carry out overall enhancements based on input be designers looks, like an evening look or a Nicole Kidman looks. One embodiment of this technique is described in the referenced provisional application.

These automatic enhancements may be done automatically or in response to user selections.

### User Input

In an embodiment, the user can specify all or part of the virtual enhancements, including modifications of automatic enhancements.

### Step 2012 in FIG. 6 - Scanning the person's real face again.

After the user has selected one or more desired virtual enhancements, the user again moves the applicator head **2**, shown in FIG. 1, over the face. The applicator head **2** again captures images of the face.

### Step 2014 in FIG. 6 - Using the global map to determine applicator head 2 location.

Software **26**, shown in FIG. 1, analyzes the skinmarks in the captured images and correlates those skinmarks with the ones stored in the global map to determine the position of the applicator head **2** relative to locations on the face for which virtual enhancements have been specified.

### Step 2016 in FIG.6 - Applying RMA on the real face to accomplish a specified enhancement.

When the software **26**, shown in FIG. 1, determines that an area of the face for which a virtual enhancement has been specified is underneath the inkjet head **8**, it instructs the inkjet head **8** to apply the RMA, contained in the cosmetic reservoir **20**, to accomplish the enhancement.

In an embodiment, the RMA can be deposited on multiple passes of the applicator head **2** over the face until the chosen enhancement is accomplished. Then no more RMA is deposited on subsequent passes.

The entire process of scanning, making virtual enhancements, and applying RMA may be repeated to the user's satisfaction.

### Other Hardware Configurations

The applicator head **2**, shown in FIG. 1, is attached to a separate computer **26** with a display **28** and controls **29**. In another embodiment, the applicator head **2** may comprise an element of an RMA applicator that itself is a computerized device with non-volatile memory, a display, and controls, so that no separate computer is required as an attachment.

### Application of Other Substances than RMAs

The applicator head **2** of the present invention may be used to apply other substances than RMAs, for example medically beneficial compounds or live skin.

### Application to Other Area than Skin

The applicator head **2** of the present invention may be used to apply RMAs and other substances to other areas than human skin. For example, it may apply substances to fabrics, fruit, walls, leaves, and flowers.

It will be apparent to those skilled in the art that different embodiments of the present invention may employ a wide range of possible hardware and of software techniques. The scope of the current invention is not limited by the specific examples described above.

## Claims

1. An applicator for an apparatus to improve the visual attractiveness of a region human skin by applying a reflectance modifying agent, the applicator comprising a computer (24) for running software (26), at least one wide angle camera (70) mounted on the applicator to identify large features on the surface of the human body; and a reflectance modifying agent applicator head (2) connected to the computer (24), the applicator head comprising
a case (4A, 4B),
an inkjet printer head (8),
a telemetric field lens (12),
at least one high resolution camera (16) mounted on the applicator head to identify small features on the surface of the human body, and
a reflectance modifying agent reservoir (20),
wherein the computer (24) is programmed by said software (26) to:
identity features of the surface,
use these features as landmarks to create a corresponding map in a computer memory,
use the map to calculate cosmetic enhancements to aspects of the surface and to determine the position the applicator for accurate deposition of the cosmetics to achieve the enhancements.

2. The applicator of claim 1 wherein computer (24) is programmed by said software (26) to capture images with one or more of the cameras (70) mounted on the applicator head to capture images of a surface of a human body by receiving exposures representing an expanding view of the area of the surface of the body from the one or more wide-angle cameras (70), the exposures providing the computer (24) with landscape data showing direction and proximity of applicator (3) to large body features.

3. The applicator of claim 2 wherein computer (24) is programmed by said software (26) to capture images with the camera (16) mounted on the applicator head to identify smaller features from an area of skin (302) under applicator head (2).

4. The applicator of claim 2 or 3 wherein said identifying features of the surface comprises analyzing the exposures from the one or more wide-angle cameras (70), in comparison with at least one generic map of large human features previously stored in computer (24) memory, to identify the location of large features.

5. The applicator of claim 3 or 4 wherein said identifying features of the surface comprises analyzing the captured images from the camera (16) mounted on the applicator head to identify smaller features, and to identify skinmarks (50, 52, 54, 56, 58, 60, 62, 63, 64, 66, 68) from those images.

6. The applicator of claim 4 or 5 wherein the computer (24) is programmed by software (26) to calculate the position of the applicator (3), in relation to the location of the large features that it has identified.

7. The applicator of claim 6 wherein the computer (24) is programmed by software (26) to track the position of the applicator head (3) relative to the skinmarks (50, 52, 54, 56, 58, 60, 62, 63, 64, 66, 68).

8. The applicator of claim 1 or 6 or 7 further comprising at least one accelerometer to measure the acceleration and track movements of the applicator (3).

9. The applicator of claims 6 and 8, wherein the computer (24) is programmed by software (26) to calculate the position of the large features and of the applicator (3) also based on rapid movements of the applicator (3) as detected by the one or more accelerometers.

10. The applicator of claims 7 and 8, wherein the computer (24) is programmed by software (26) to calculate the position of the skinmarks and of the applicator head (2) based on rapid movements of the applicator (3) as detected by the one or more accelerometers.

11. The applicator of any one of claims 7-10 wherein the computer (24) is programmed by software (26) to create said map in the computer memory by using the large features and the skinmarks it has identified relative to the position of the applicator (3), optionally wherein the computer (24) is programmed by software (26) to store the global map either automatically at periodic intervals or each time the applicator head (2) is raised from the surface being scanned.

12. The applicator of claim 1 or 11, wherein the cosmetic enhancements to aspects of features automatically calculated and, optionally, said cosmetic enhancements being displayed on a display (28) connected to said computer (24).

13. The applicator of claim 1 or 11 wherein the computer (24) is programmed by software (26) to determine again the position of the applicator relative to the actual features after the calculation of cosmetic enhancements, by:
capturing again images of the surface by said wide-angle camera 70 and high-resolution camera (16);
identifying large features and skinmarks of the scanned surface and the position of the applicator (3) relative to landmarks and skinmarks in the global map of the scanned surface;
calculating the position of the applicator (3) relative to actual features.

14. The applicator of claim 13, wherein calculating the position of the applicator (3) relative to the actual features is based on detection of rapid movements of applicator (3) made by one or more accelerometers (72).

15. The applicator of claims 13 or 14, wherein the computer (24) is programmed by software (26) to
determine that an area of skin or other feature for which an enhancement has been specified is underneath the inkjet head (8), and
instruct the inkjet head (8) to apply the RMA, contained in the cosmetic reservoir (20), to accomplish the enhancement.

16. The applicator of claim 1 wherein the at least one plurality of wide-angle cameras mounted on the applicator head to capture images of a surface of a human body further comprises
six miniature wide-angle cameras, outwardly and downwardly aimed relative to the case, such that each wide-angle of the six miniature cameras has a field of view that is approximately 60° of a visual area below and around the applicator.

17. The applicator of any preceding claim, wherein the reflectance modifying agent applicator head (2) is sized to fit in intimate contact across a human face.

## Patentansprüche

1. Applikator für eine Vorrichtung zur Verbesserung der visuellen Attraktivität einer Zone menschlicher Haut durch Anwendung eines Mittels zur Modifikation des Reflexionsgrads, wobei der Applikator einen Computer (24) zum Ausführen von Software (26),
mindestens eine Weitwinkelkamera (70), die an dem Applikator montiert ist, um große Merkmale an der Oberfläche des menschlichen Körpers zu identifizieren, und
einen Applikatorkopf (2) für das Mittel zur Modifikation des Reflexionsgrads, der mit dem Computer (24) verbunden ist, umfasst, wobei der Applikatorkopf umfasst:
ein Gehäuse (4A, 4B),
einen Tintenstrahl-Druckkopf (8),
eine Telemetriefeldlinse (12),
mindestens eine hochauflösende Kamera (16), die an dem Applikatorkopf montiert ist, um kleine Merkmale an der Oberfläche des menschlichen Körpers zu identifizieren, und
ein Reservoir (20) für das Mittel zur Modifikation des Reflexionsgrads,
wobei der Computer (24) durch die Software (26) programmiert ist:
Merkmale der Oberfläche zu identifizieren,
diese Merkmale als Orientierungspunkte zu verwenden, um eine entsprechende Karte in einem Computerspeicher zu erzeugen,
die Karte zu verwenden, um kosmetische Optimierungen an Aspekten der Oberfläche zu berechnen und um die Position des Applikators zum genauen Aufbringen der Kosmetika zu bestimmen, um die Optimierungen zu erzielen.

2. Applikator nach Anspruch 1, wobei der Computer (24) durch die Software (26) programmiert ist, Bilder mit einer oder mehreren der Kameras (70) aufzunehmen, die an dem Applikatorkopf montiert sind, um Bilder einer Oberfläche eines menschlichen Körpers aufzunehmen, indem Aufnahmen empfangen werden, die eine vergrößerte Ansicht des Bereichs der Oberfläche des Körpers von der einen oder den mehreren Weitwinkelkameras (70) darstellen, wobei die Aufnahmen für den Computer (24) Landschaftsdaten bereitstellen, welche die Richtung und die Nähe des Applikators (3) zu großen Körpermerkmalen zeigen.

3. Applikator nach Anspruch 2, wobei der Computer (24) durch die Software (26) programmiert ist, Bilder mit den Kameras (16) aufzunehmen, die an dem Applikatorkopf montiert sind, um kleinere Merkmale aus einem Bereich der Haut (302) unter dem Applikatorkopf (2) zu identifizieren.

4. Applikator nach Anspruch 2 oder 3, wobei die Identifikation von Merkmalen der Oberfläche eine Analyse der Aufnahmen von der einen oder den mehreren Weitwinkelkameras (70) umfasst, verglichen mit mindestens einer generischen Karte großer menschlicher Merkmale, die in dem Computer (24) vorher gespeichert wurden, um den Ort großer Merkmale zu identifizieren.

5. Applikator nach Anspruch 3 oder 4, wobei die Identifikation von Merkmalen der Oberfläche eine Analyse der aufgenommenen Bilder von der Kamera (16) umfasst, die an dem Applikatorkopf montiert ist, um kleinere Merkmale zu identifizieren, und um Hautmarkierungen (50, 52, 54, 56, 58, 60, 62, 63, 64, 66, 68) aus diesen Bildern zu identifizieren.

6. Applikator nach Anspruch 4 oder 5, wobei der Computer (24) durch Software (26) programmiert ist, die Position des Applikators (3) in Bezug auf den Ort der großen Merkmale zu berechnen, die sie identifiziert hat.

7. Applikator nach Anspruch 6, wobei der Computer (24) durch Software (26) programmiert ist, die Position des Applikatorkopfs (3) relativ zu den Hautmarkierungen (50, 52, 54, 56, 58, 60, 62, 63, 64, 66, 68) zu verfolgen.

8. Applikator nach Anspruch 1 oder 6 oder 7, ferner umfassend mindestens ein Akzelerometer, um die Beschleunigungs- und Verfolgungsbewegungen des Applikators (3) zu messen.

9. Applikator nach Anspruch 6 und 8, wobei der Computer (24) durch Software (26) programmiert ist, die Position der großen Merkmale und des Applikators (3) auch auf der Basis rascher Bewegungen des Applikators (3) zu berechnen, wie durch das eine oder die mehreren Akzelerometer detektiert.

10. Applikator nach Anspruch 7 und 8, wobei der Computer (24) durch Software (26) programmiert ist, die Position der Hautmarkierungen und des Applikatorkopfs (2) auf der Basis rascher Bewegungen des Applikators (3) zu berechnen, wie durch das eine oder die mehreren Akzelerometer detektiert.

11. Applikator nach einem der Ansprüche 7 bis 10, wobei der Computer (24) durch Software (26) programmiert ist, die Karte in dem Computerspeicher unter Verwendung der großen Merkmale und der Hautmarkierungen, die sie identifiziert hat, relativ zu der Position des Applikators (3) zu erzeugen, wobei gegebenenfalls der Computer (24) durch Software (26) programmiert ist, die globale Karte entweder automatisch in periodischen Intervallen oder jedes Mal zu speichern, wenn der Applikatorkopf (2) von der Oberfläche, die gescannt wird, abgehoben wird.

12. Applikator nach Anspruch 1 oder 11, wobei die kosmetischen Optimierungen an Aspekten von Merkmalen automatisch berechnet werden und gegebenenfalls die kosmetischen Optimierungen auf einer Anzeige (28) angezeigt werden, die mit dem Computer (24) verbunden ist.

13. Applikator nach Anspruch 1 oder 11, wobei der Computer (24) durch Software (26) programmiert ist, die Position des Applikators relativ zu den tatsächlichen Merkmalen nach der Berechnung kosmetischer Optimierungen erneut zu bestimmen durch:
erneute Aufnahme von Bildern der Oberfläche durch die Weitwinkelkamera (70) und hochauflösende Kamera (16);
Identifikation von großen Merkmalen und Hautmarkierungen der gescannten Oberfläche und der Position des Applikators (3) relativ zu Orientierungspunkten und Hautmarkierungen in der globalen Karte der gescannten Oberfläche;
Berechnung der Position des Applikators (3) relativ zu tatsächlichen Merkmalen.

14. Applikator nach Anspruch 13, wobei die Berechnung der Position des Applikators (3) relativ zu den tatsächlichen Merkmalen auf der Detektion rascher Bewegungen des Applikators (3) basiert, die durch ein oder mehrere Akzelerometer (72) vorgenommen werden.

15. Applikator nach Anspruch 13 oder 14, wobei der Computer (24) durch Software (26) programmiert ist:
zu bestimmen, dass ein Bereich der Haut oder eines anderen Merkmals, für den eine Optimierung spezifiziert wurde, unterhalb des Tintenstrahlkopfs (8) liegt; und
den Tintenstrahlkopf (8) anzuweisen, das RMA, das in dem kosmetischen Reservoir (20) enthalten ist, aufzubringen, um die Optimierung zu erzielen.

16. Applikator nach Anspruch 1, wobei die mindestens eine von den mehreren Weitwinkelkameras, die an dem Applikatorkopf montiert sind, um Bilder einer Oberfläche eines menschlichen Körpers aufzunehmen, ferner umfasst:
sechs Miniatur-Weitwinkelkameras, die nach außen und nach unten relativ zu dem Gehäuse weisen, so dass jeder Weitwinkel der sechs Miniaturkameras ein Sichtfeld aufweist, das ungefähr 60° eines Sichtbereichs unter dem und rund um den Applikator beträgt.

17. Applikator nach einem der vorhergehenden Ansprüche, wobei der Applikatorkopf (2) für das Mittel zur Modifikation des Reflexionsgrads bemessen ist, in einem engen Kontakt quer über ein menschliches Gesicht zu passen.

## Revendications

1. Applicateur pour un dispositif destiné à améliorer l'attrait visuel de la peau humaine d'une région par application d'un agent modifiant le coefficient de réflexion, l'applicateur comprenant
un ordinateur (24) pour exécuter un logiciel (26), au moins une caméra grand angle (70) montée sur l'applicateur, pour identifier de grands traits sur la surface du corps humain, et
une tête d'applicateur d'agent modifiant le coefficient de réflexion (2) connectée à l'ordinateur (24), la tête d'applicateur comprenant
un boîtier (4A, 4B),
une tête d'imprimante à jet d'entre (8),
une lentille de champ télémétrique (12),
au moins une caméra haute définition (16) montée sur la tête d'applicateur pour identifier des traits petits sur la surface du corps humain, et
un réservoir d'agent modifiant le coefficient de réflexion (20),
dans lequel l'ordinateur (24) est programmé par ledit logiciel (26) pour :
identifier des traits de la surface,
utiliser ces traits comme des points de repère pour créer une carte correspondante dans une mémoire d'ordinateur,
utiliser la carte pour calculer des améliorations cosmétiques à des aspects de la surface et pour déterminer la position de l'applicateur pour une déposition précise des cosmétiques afin que ces améliorations soient réalisées.

2. Applicateur selon la revendication 1, dans lequel l'ordinateur (24) est programmé par ledit logiciel (26) pour capturer des images avec une ou plusieurs des caméras (70) montées sur la tête d'applicateur pour capturer des images d'une surface d'un corps humain par réception d'expositions représentant une vue agrandie de la zone de la surface du corps provenant de la ou des caméras grand angle (70), les expositions donnant à l'ordinateur (24) des données de panorama montrant la direction et la proximité de l'applicateur (3) par rapport à de grands traits du corps.

3. Applicateur selon la revendication 2, dans lequel l'ordinateur (24) est programmé par ledit logiciel (26) pour capturer des images avec la caméra (16) montée sur la tête d'applicateur pour identifier des traits plus petits dans la zone de la peau (302) sous la tête d'applicateur (2).

4. Applicateur selon la revendication 2 ou 3, dans lequel ladite identification de traits de la surface comprend l'analyse des expositions provenant de la ou des caméras grand angle (70), en comparaison avec au moins une carte générique de grands traits humains préalablement stockée dans la mémoire d'ordinateur (24), pour identifier l'emplacement de grands traits.

5. Applicateur selon la revendication 3 ou 4, dans lequel ladite identification de traits de la surface comprend l'analyse des images capturées par la caméra (16) montée sur la tête d'applicateur pour identifier des traits plus petits, et pour identifier des marques cutanées (50, 52, 54, 56, 58, 60, 62, 63, 64, 66, 68) à partir de ces images.

6. Applicateur selon la revendication 4 ou 5, dans lequel l'ordinateur (24) est programmé par le logiciel (26) pour calculer la position de l'applicateur (3), en relation avec l'emplacement des grands traits qu'il a identifiés.

7. Applicateur selon la revendication 6, dans lequel l'ordinateur (24) est programmé par le logiciel (26) pour pister la position de la tête d'applicateur (3) par rapport aux marques cutanées (50, 52, 54, 56, 58, 60, 62, 63, 64, 66, 68).

8. Applicateur selon la revendication 1, 6 ou 7, comprenant en outre au moins un accéléromètre pour mesurer l'accélération et suivre les mouvements de l'applicateur (3).

9. Applicateur selon les revendications 6 et 8, dans lequel l'ordinateur (24) est programmé par le logiciel (26) pour calculer la position des grands traits et de l'applicateur (3) également sur la base de mouvements rapides de l'applicateur (3) tels que détectés par le ou les accéléromètres.

10. Applicateur selon les revendications 7 et 8, dans lequel l'ordinateur (24) est programmé par le logiciel (26) pour calculer la position des marques cutanées et de la tête d'applicateur (2) sur la base de mouvements rapides de l'applicateur (3) tels que détectés par le ou les accéléromètres.

11. Applicateur selon l'une quelconque des revendications 7 à 10, dans lequel l'ordinateur (24) est programmé par le logiciel (26) pour créer ladite carte dans la mémoire d'ordinateur en utilisant les grands traits et les marques cutanées qu'il a identifiés en relation avec la position de l'applicateur (3), éventuellement dans lequel l'ordinateur (24) est programmé par le logiciel (26) pour stocker la carte globale soit automatiquement à intervalles périodiques soit à chaque fois que la tête d'applicateur (2) est relevée de la surface qui est balayée.

12. Applicateur selon la revendication 1 ou 11, dans lequel les améliorations cosmétiques à des aspects de traits sont automatiquement calculées et, éventuellement, lesdites améliorations cosmétiques sont affichées sur un affichage (28) connecté audit ordinateur (24).

13. Applicateur selon la revendication 1 ou 11, dans lequel l'ordinateur (24) est programmé par le logiciel (26) pour déterminer de nouveau la position de l'applicateur par rapport aux traits réels après le calcul des améliorations cosmétiques, par :
de nouveau capture d'images de la surface par ladite caméra grand angle (70) et la caméra haute définition (16) ;
identification de grands traits et de marques cutanées de la surface balayée et de la position de l'applicateur (3) par rapport aux points de repère et aux marques cutanées dans la carte globale de la surface balayée ;
calcul de la position de l'applicateur (3) par rapport aux traits réels.

14. Applicateur selon la revendication 13, dans lequel le calcul de la position de l'applicateur (3) par rapport aux traits réels est basé sur la détection de mouvements rapides de l'applicateur (3) effectuée par un ou plusieurs accéléromètres (72).

15. Applicateur selon la revendication 13 ou 14, dans lequel l'ordinateur (24) est programmé par le logiciel (26) pour
déterminer qu'une zone de la peau ou un autre trait pour lequel une amélioration a été spécifiée se trouve sous la tête de jet d'encre (8), et
donner pour instruction à la tête de jet d'encre (8) d'appliquer l'agent modifiant le coefficient de réflexion contenu dans le réservoir de cosmétique (20) pour accomplir l'amélioration.

16. Applicateur selon la revendication 1, dans lequel l'au moins une pluralité de caméras grand angle montée sur la tête d'applicateur pour capturer des images d'une surface d'un corps humain comprend en outre six caméras grand angle miniatures, dirigées vers le haut et vers le bas par rapport au boîtier, de façon que chaque grand angle des six caméras miniatures ait un champ de vision représentant environ 60° d'une zone visuelle sous et autour de l'applicateur.

17. Applicateur selon l'une quelconque des revendications précédentes, dans lequel la tête d'applicateur d'agent modifiant le coefficient de réflexion (2) est dimensionnée pour s'ajuster en contact intime sur un visage humain.
